(19) Europäisches Patentamt
European Patent Office
Office européen des brevets

(11) Veröffentlichungsnummer: **0 000 390**
**B1**

(12) **EUROPÄISCHE PATENTSCHRIFT**

(45) Veröffentlichungstag der Patentschrift: **14.10.81**

(21) Anmeldenummer: **78100347.0**

(22) Anmeldetag: **11.07.78**

(51) Int. Cl.³: **C 07 C 119/16,**
C 07 C 129/12,
C 07 C 153/057,
C 07 C 161/00

# (54) Derivate der 2-(2,2-Dihalogenvinyl)-3,3-dimethyl-Cyclopropancarbonsäure und Verfahren zu ihrer Herstellung.

(30) Priorität: **16.07.77 DE 2732213**

(43) Veröffentlichungstag der Anmeldung:
**24.01.79 Patentblatt 79/2**

(45) Bekanntmachung des Hinweises auf die Patenterteilung:
**14.10.81 Patentblatt 81/41**

(84) Benannte Vertragsstaaten:
**BE CH DE FR GB NL**

(56) Entgegenhaltungen:
**DE - A - 2 621 832**
**DE - A - 2 639 777**
**DE - A - 2 751 610**

(73) Patentinhaber: **BAYER AG**
***Zentralbereich Patente, Marken und Lizenzen***
**D-5090 Leverkusen 1, Bayerwerk (DE)**

(72) Erfinder: **Arlt, Dieter, Dr.**
**Rybnikerstrasse 2**
**D-5000 Köln 80 (DE)**
Erfinder: **Jautelat, Manfred, Dr**
**Buchenweg 18**
**D-5093 Burscheid 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 390 B1

# Derivate der 2-(2,2-Dihalogenvinyl)-3,3-dimethylcyclopropancarbonsäure, und Verfahren zu ihrer Herstellung

Die vorliegende Erfindung betrifft neue Derivate der 2-(2,2-Dihalogenvinyl)-3,3-dimethylcyclopropancarbonsäure, ein Verfahren zu ihrer Herstellung und Zwischenprodukte zu ihrer Herstellung.

Die Herstellung von 2-(2,2-Dichlorvinyl)-3,3-dimethylcyclopropancarbonsäure durch Umsetzung von 1,1-Dichlor-4-methyl-1,3-pentadien mit Diazoessigester und nachfolgender Hydrolyse ist bereits bekannt (Farkas et al, Collect. Czechosl. Chem. Commun. *24*, 2250 (1959)). Dieses Verfahren weist jedoch eine Reihe von Nachteilen auf. So ist die Handhabung großer Mengen Diazoessigester, der sich explosionsartig zersetzen kann, in der Technik mit großen Risiken behaftet. Auch aus physiologischer Sicht ist die Verwendung von Diazoverbindungen problematisch.

Es ist ferner vorgeschlagen worden, anstelle der Carbonsäure 1-Cyano-2-(2,2-dichlorvinyl)-3,3-dimethyl-cyclopropan als Zwischenstufe zur Herstellung der insektizid wirksamen Carbonsäureester zu verwenden. Dieses Nitril läßt sich durch Wasserabspaltung aus dem entsprechenden Aldoxim (vgl. Deutsche Offenlegungsschrift 2 621 832) oder durch basenkatalysierten Ringschluß aus 3-Brom-1-cyano-2,2-dimethyl-5,5,5-trichlor-pentan (vgl. deutsche Offenlegungsschrift 2 261 831) herstellen. Die Verfahren leiden jedoch unter dem Mangel, daß 1-Cyano-2-(2,2-dichlorvinyl)-3,3-dimethyl-cyclopropan selbst unter drastischen Bedingungen nur in geringen Ausbeuten zur entsprechenden Carbonsäure verseift werden kann.

Aus DE—OS 2 639 777 sind 2-(ββ-disubstituiertes Vinyl)-3,3-dimethylcyclopropancarbonsäure-amid sowie -N-alkylamide bekannt. Sie werden in einem mehrstufigen Verfahren aus den entsprechenden 2-Acetyl-3,3-dimethylcyclopropancarbonsäureamiden erhalten. Dieses Verfahren ist technisch aufwendig.

Es werden nun die neuen Derivate der 2-(2,2-Dihalogenvinyl)-3,3-dimethylcyclopropancarbonsäure der allgemeinen Formel (I)

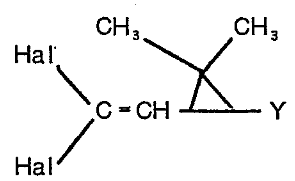

(I)

in welcher
Hal unabhängig voneinander für Halogen steht und
Y für ein C-Atom steht, das ausschließlich sowohl einfach als auch doppelt an Sauerstoff oder Schwefel und/oder Stickstoff gebunden ist, wobei mindestens eine Bindung durch Stickstoff besetzt ist, und Y nicht für den Carbonamidrest, steht,
gefunden.

Es wurde ferner gefunden, daß die neuen Derivate der 2-(2,2-Dihalogenvinyl)-3,3-dimethylcyclopropancarbonsäure der allgemeinen Formel (I) hergestellt werden können, indem man eine Verbindung der allgemeinen Formel (II)

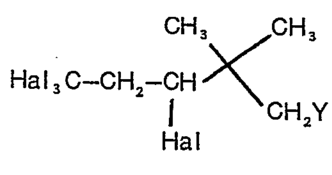

(II)

in welcher
Hal und Y die oben angegebene Bedeutung haben,
in Gegenwart eines Verdünnungsmittels mit einer Base behandelt.

Die Cyclopropancarbonsäurederivate der allgemeinen Formel (I) dienen als Zwischenprodukte zur Herstellung der ihnen zugrundeliegenden bekannten Cyclopropancarbonsäuren, die ihrerseits Zwischenprodukte zur Herstellung bekannter Insektizide sind. Die Cyclopropancarbonsäuren werden erhalten, indem man die Verbindungen der allgemeinen Formel (I) sauer oder alkalisch unter geeigneten Bedingungen hydrolysiert (Houben-Weyl, Methoden der organischen Chemie, Band VIII, S. 432 (1952) und Methodicum Chimicum, Band 5, S. 572 (1975)).

Die Verwendung der Cyclopropancarbonsäurederivate der allgemeinen Formel (I) stellt eine entscheidende Verbesserung des Verfahrens zur Herstellung von Cyclopropancarbonsäuren, ausgehend vom entsprechenden Cyclopropancarbonsäurenitril, dar. Es wird dabei die Stufe des nur schwer und in geringer Ausbeute zur Cyclopropancarbonsäure oder zu deren Derivaten zu verseifenden Cyclopropancarbonsäurenitrils umgangen.

Es ist als ausgesprochen überraschend zu bezeichnen, daß die Umwandlung der Cyangruppe in

funktionelle Carbonsäurederivate auf der Stufe des 4-Cyano-3,3-dimethyl-1-butens, des Ausgangsproduktes zur Herstellung der Verbindungen der Formel (I), sehr viel leichter und besser möglich ist als auf der Stufe des 1-Cyano-2-(2,2-dichlorvinyl)-3,3-dimethylcyclopropans. Die Verwendung der erfindungsgemäßen neuen Stoffe führt zu einem Verfahren, das den entscheidenden Vorteil aufweist, daß die Synthese der insektizid wirksamen 2-(2,2-Dihalogenvinyl)-3,3-dimethylcyclopropancarbonsäureester wie beispielsweise 2-(2,2-Dichlorvinyl)-3,3-dimethylcyclopropancarbonsäure-3-phenoxybenzylester einfacher und in besseren Ausbeuten möglich ist als aus den entsprechenden Nitrilen.

Die Ausgangsstoffe zur Herstellung der Cyclopropancarbonsäurederivate der Formel (I) sind neu, sie sind durch die Formel (II) allgemein definiert. In der Formel (II) steht Hal bevorzugt für Chlor oder Brom und Y bevorzugt für einen der folgenden Reste a bis d:

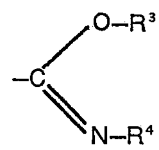

(a)

wobei

$R^3$, $R^4$ unabhängig voneinander für Wasserstoff, Alkyl, Aryl oder Acyl stehen, oder gemeinsam mit den angrenzenden Atomen einen Heterocyclus bilden können, ferner für

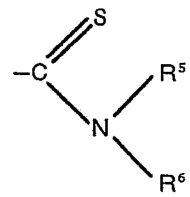

(b)

wobei

$R^5$, $R^6$ unabhängig voneinander für Wasserstoff, Aryl, Hydroxy, Alkoxy oder Amino stehen, oder gemeinsam mit dem angrenzenden N-Atom einen Heterocyclus bilden können, ferner für

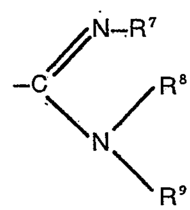

(c)

wobei

$R^7$ für Wasserstoff, Alkyl, Aryl, Amino, Alkylamino, Dialkylamino, hydroxyl, Alkoxy steht und

$R^8$, $R^9$ unabhängig voneinander für Wasserstoff, Alkyl, Aryl, Arylamino, Dialkylamino oder Hydroxyl stehen oder gemeinsam zwei der Reste $R^7$, $R^8$ und $R^9$ mit den angrenzenden Atomen einen Heterocyclus bilden können, ferner für

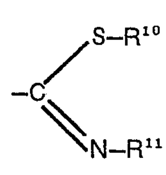

(d)

wobei

$R^{10}$ für Alkyl steht und

$R^{11}$ für Wasserstoff, Alkyl, Aryl, Hydroxyl, Alkoxy oder Amino steht oder $R^{10}$ und $R^{11}$ gemeinsam mit den angrenzenden Atomen einen Heterocyclus bilden können.

Besonders bevorzugt sind Ausgangsverbindungen der allgemeinen Formel (II), in der Y für den Rest der Formel (a) steht, wobei $R^3$ für Alkylreste mit 1 bis 4 C-Atomen, Aryl wie Phenyl, oder Acyl mit 1 bis 7 C-Atomen steht und $R^4$ für Wasserstoff, Alkylreste mit 1 bis 4 C-Atomen, Aryl wie Phenyl oder Acylreste mit 1 bis 7 C-Atomen steht und $R^3$ und $R^4$ gemeinsam für einen Alkylenrest mit 2 bis 5 C-Atomen wie Ethylen oder Trimethylen stehen, ferner für den Rest der Formel (b) steht, wobei $R^5$ für Wasserstoff, Alkylreste mit 1 bis 4 C-Atomen steht und $R^6$ für Wasserstoff, Alkylreste mit 1 bis 4 C-Atomen, Aryl wie Phenyl, Hydroxyl, Alkoxyreste mit 1 bis 4 C-Atomen oder Amino steht und $R^5$ und $R^6$ auch für Teile eines Ringes wie Tetramethylen, Pentamethylen oder $-CH_2-CH_2-O-CH_2-CH_2-$ stehen, ferner für den Rest der Formel (c) steht, wobei $R^7$ für Wasserstoff, Alkylreste mit 1 bis 4 C-Atomen, Aryl wie Phenyl, Alkylamino mit 1 bis 4 C-Atomen, Dialkylamino mit 2 bis 8 C-Atomen, Hydroxyl, Alkoxyreste mit 1 bis 4 C-Atomen steht und $R^8$ für Wasserstoff und Alkylreste mit 1 bis 4 C-Atomen steht und $R^9$ für Wasserstoff, Alkylreste mit 1 bis 4 C-Atomen, Aryl wie Phenyl, Amino, Alkylamino mit 1 bis 4 C-Atomen, Dialkylamino mit 2 bis 8 C-Atomen oder Hydroxyl steht und $R^7$ und $R^8$ gemeinsam für einen Alkylenrest mit 2 bis 5 C-Atomen wie Ethylen oder Trimethylen stehen und $R^8$ und $R^9$ auch für Teile eines Ringes wie Tetramethylen, Pentamethylen oder $-CH_2-CH_2-O-CH_2-CH_2-$ stehen, ferner für den Rest der Formel (d) steht, wobei $R^{10}$ für Alkylreste mit 1 bis 4 C-Atomen steht, $R^{11}$ für Wasserstoff, Alkylreste mit 1 bis 4 C-Atomen, Aryl wie Phenyl, Hydroxyl, Alkoxyresten mit 1 bis 4 C-Atomen oder Amino steht.

Nach einer besonderen Ausführungsform dieser Erfindung lassen sich die einzelnen Reaktionsstufen in einem Verfahren so zusammenfassen, daß auf die Reinigung und Isolierung der reinen Zwischenprodukte verzichtet werden kann.

Als Beispiele für Ausgangsverbindungen der allgemeinen Formel (II), die besonders vorteilhaft zur Herstellung der Cyclopropancarbonsäurederivate verwendet werden können, seien genannt:

3,3-Dimethyl-4,6,6,6-tetrachlorhexansäure-imid-ethylester
3,3-Dimethyl-4,6,6,6-tetrachlorhexansäure-methylimidmethylester
2-(2',2'-Dimethyl-3',5',5',5'-tetrachlorpentyl)oxazolin
2-(2',2'-Dimethyl-3',5',5',5'-tetrabrompentyl)oxazolin
2-(2',2'-Dimethyl-3',5',5',5'-tetrachlorpentyl-5,6-dihydro-4H-1,3-oxazin
3,3-Dimethyl-4,6,6,6-tetrachlorhexanhydroximsäure-O-methylethylester
3,3-Dimethyl-4,6,6,6-tetrachlorhexanthiocarbonsäureamid
3,3-Dimethyl-4,6,6,6-tetrachlorhexanthiocarbonsäure-N-tert.-butylamid
3,3-Dimethyl-4,6,6,6-tetrachlorhexanthiocarbonsäure-N-ethylamid
3,3-Dimethyl-4,6,6,6-tetrachlorhexansäure-amidin
3,3-Dimethyl-4,6,6,6-tetrachlorhexansäure-N-methyl-N'-phenylamidin
3,3-Dimethyl-4,6,6,6-tetrachlorhexansäure-amidoxim
3,3-Dimethyl-4,6,6,6-tetrachlorhexansäure-amidrazon
3,3-Dimethyl-4,6,6,6-tetrachlorhexanthiocarbonsäure-N-methylhydrazonid-S-methylester
3,3-Dimethyl-4,6,6,6-tetrachlorhexan-S-methyl-thiohydroxamsäure.

Wie bereits erwähnt, sind die Ausgangsverbindungen der allgemeinen Formel (II) neu. Sie können jedoch erhalten werden, indem man Verbindungen der Formel (III)

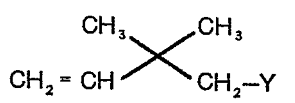

(III)

in welcher

Y die oben angegebene Bedeutung hat, gegebenenfalls in Anwesenheit eines Verdünnungsmittels mit Tetrahalogenmethan in Gegenwart eines Katalysators umsetzt.

Dabei kann der Reaktionsablauf durch folgendes Formel-schema wiedergegeben werden:

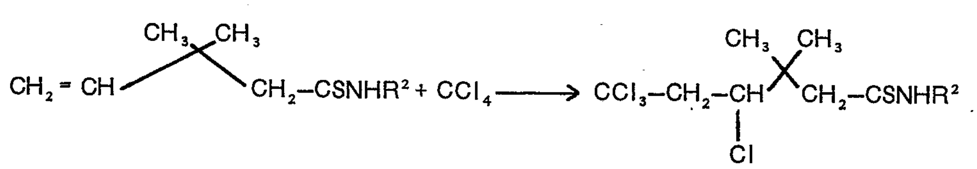

Besonders bevorzugte Tetrahalogenmethane sind Tetrachlorkohlenstoff, Bromtrichlormethan oder Tetrabrommethan.

Bevorzugte Verbindung der allgemeinen Formel (III) sind diejenigen, die zu den bevorzugten Verbindungen der Formel (II) führen.

Die Addition der Tetrahalogenmethane an die Verbindungen der Formel (III) wird in Temperaturbereich von 50 bis 120°C, vorzugsweise 70 bis 100°C, unter Verwendung von Katalysatoren wie Dibenzoylperoxid, Azobisisobuttersäurenitril, Kupfer- oder Eisensalzen durchgeführt.

Als Verdünnungsmittel eignen sich alle inerten organischen Lösungsmittel wie Kohlenwasserstoffe, Ether, Nitrile, Ester, Ketone, Bevorzugt werden jedoch die auch als Ausgangsstoffe verwendbaren Tetrahalogenmethane verwendet.

Die Ausgangsverbindungen der allgemeinen Formel III sind zum Teil neu. Sie können erhalten werden, indem man

a) die Nitrilgruppe von 4-Cyano-3,3-dimethyl-1-buten in an sich bekannter Weise in den Rest Y der Verbindungen der allgemeinen Formel III überführt (vgl. Houben Weyl, Methoden der Organischen Chemie Band VIII und Methodicum Chimicum, Band 6, 1974, Thieme Verlag Stuttgart) oder
b) den Carboxylrest der 3,3-Dimethyl-4-pentensäure in an sich bekannter Weise in den Rest Y der Verbindungen der allgemeinen Formel III überführt (vgl. Houben Weyl, Methoden der Organischen Chemie Band VIII und Methodicum Chimicum, Band 6, 1974, Thieme Verlag Stuttgart).

Das Verfahren zur Herstellung der erfinungsgemäßen 2(2,2-Dihalogenvinyl)-3,3-dimethylcyclopropancarbonsäurederivate wird durch Einwirkung von wenigstens zwei Mol Base auf die Verbindungen der Formel II durchgeführt und ergibt unter Cyclisierung und β-Eliminierung die erfindungsgemäßen Verbindungen. Dabei können Cyclisierung und β-Eliminierung sowohl nacheinander als auch gleichzeitig erfolgen.

Als Basen kommen tertiäre Amine, beispielsweise Pyridin, Triäthylamin, Dimethylanilin, Benzyldimethylamin, N-Methylpiperidin, 1,8-Diazabicyclo(5,4,0)-undecen, Alkalimetallalkolate, beispielsweise Natriummethylat, Natriumäthylat, Kalium-t-butylat und ebenso Alkalihydroxide wie Natriumhydroxid oder Kaliumhydroxid sowie Alkali- und Erdalkalicarbonate.

Als Verdünnungsmittel eignen sich Alkohole wie Methanol, Äthanol oder t-Butanol, Äther wie Dioxan oder Diäthyläther, und polare Lösungsmittel wie Acetonitril, Dimethylformamid, Dimethylsulfoxid oder Hexamethylphosphorsäuretriamid.

Die Reaktionstemperaturen können in einem größeren Bereich variiert werden. Im allgemeinen arbeitet man zwischen Raumtemperatur und 120°C, bevorzugt zwischen 40° und 80°C.

Verbindungen der allgemeinen Formel III

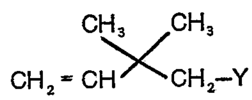

(III)

in welcher

Y für den Oxazolinrest der allgemeinen Formel IV steht

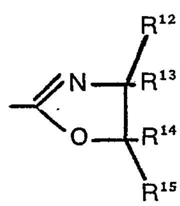

in welcher

$R^{12}$—$R^{15}$ unabhängig voneinander für Wasserstoff, Alkyl, Aralkyl, Aryl stehen oder gemeinsam mit den angrenzenden C-Atomen einen carbocylischen Ring bilden können

sind neu.

Man erhält sie, indem man 4-Cyano-3,3-dimethyl-1-buten der Formel V

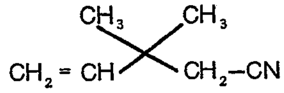

(V)

mit 2-Amino-alkanolen der allgemeinen Formel VI

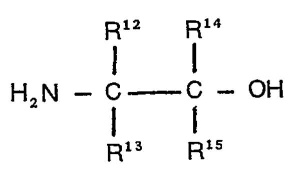

in welcher

$R^{12}$—$R^{15}$ die oben angegebene Bedeutung haben, bei Temperaturen von 150—200°C gegebenenfalls in Gegenwart eines Katalysators sowie eines Verdünnungsmittels umsetzt.

Bevorzugt sind solche der neuen Verbindungen der allgemeinen Formel III in denen die Reste $R^{12}$—$R^{15}$ de Oxazolinrestes der allgemeinen Formel IV unabhängig voneinander für Wasserstoff, Alkyl mit 1—4 C-Atomen, Aralkyl mit 7—9 C-Atomen oder Phenyl, das ggf. durch Halogen, Alkoxy oder Phenoxy substituiert ist stehen, wobei $R^{12}$ und $R^{13}$ oder/sowie $R^{14}$ und $R^{15}$ eine Alkylenkette mit 4—6 C-Atomen bilden können und $R^{12}$ mit $R^{14}$ oder $R^{15}$ eine Alkylenkette mit 3—4 C-Atomen bilden kann.

Als Beispiele seien im einzelnen genannt:

2-(2,2-Dimethyl-3-buten-1-yl)-2-oxazolin,
2-(2,2-Dimethyl-3-buten-1-yl)-4,4-dimethyl-2-oxazolin
2-(2,2-Dimethyl-3-buten-1-yl)-5,5-dimethyl-2-oxazolin
2-(2,2-Dimethyl-3-buten-1-yl)-4,4,5,5-tetramethyl-2-oxazolin
2-(2,2-Dimethyl-3-buten-1-yl)-5-benzyl-2-oxazolin
2-(2,2-Dimethyl-3-buten-1-yl)-4,4-tetramethylen-2-oxazolin
2-(2,2-Dimethyl-3-buten-1-yl)-4,5-tetramethylen-2-oxazolin
2-(2,2-Dimethyl-3-buten-1-yl)-4-phenyl-2-oxazolin
2-(2,2-Dimethyl-3-buten-1-yl)-5-phenyl-2-oxazolin
2-(2,2-Dimethyl-3-buten-1-yl)-5-(m-phenoxyphenyl)-2-oxazolin

Das Verfahren zur Herstellung der neuen Verbindungen der Formel III wird analog dem in Angew. Chem. Band 88, Seite 321 (1976) beschriebenen Verfahren durchgeführt. Als Katalysatoren werden dabei bevorzugt verwendet:

Cadmiumacetate, Zinkacetat, Manganacetat, Zinkchlorid, Kupfer-(II)-chlorid, Cobaltacetat oder Lithiumchlorid.

Die vorliegende Erfindung wird durch die folgenden Beispiele erläutert. Dabei wird unter .1 eines jeden Beispiels die Bildung des Restes Y, d.h. der Carbonsäurederivatgruppe, ausgehend von 4-Cyano-3,3-dimethyl-1-oder 3,3-Dimethyl-4-pentensäure, beschrieben.

Unter .2 eines jeden Beispiels wird die Addition des Tetrahalogenmethans an die Doppelbindung des Ausgangsstoffes, der gemäß den unter .1 beschriebenen Verfahren erhalten wird, beschrieben.

Unter .3 eines jeden Beispiels wird schließlich die Ringbindung und $\beta$-Eliminierung aus den gemäß .2 erhaltenen Produkten beschrieben.

## Beispiel 1

Herstellung von 2-(2'-(2,2-Dichlorvinyl)-3',3'-dimethylcyclopropyl)-oxazolin

1.1 10,9 g (0,1 Mol) 4-Cyano-3,3-dimethyl-1-buten werden mit 6,7 g (0,11 Mol) Aminoethanol unter Katalyse von Cadmiumacetat bis zum Ende der $NH_3$-Entwicklung auf 150 bis 160°C erhitzt. Nach dem Erkalten wird das Reaktionsgemisch in Tetrachlorkohlenstoff aufgenommen.

1.2 Diese Lösung des 2-(2',2'-Dimethyl-3'-butenyl)-oxazolins in $CCl_4$ wird unter Zusatz von 0,5 g Dibenzoylperoxid 15 Stunden unter Rückfluß erhitzt. Das Lösungsmittel wird im Vakuum abgezogen und der Rückstand in 100 ml DMSO gelöst.

1.3 Nach Zusatz von 24 g (0,21 Mol) Kalium-t-butylat erhitzt man 1 Stunde auf 70°C. Die Lösung wird mit Methylenchlorid verdünnt und mit Wasser mehrfach ausgeschüttelt. Nach Trocknen und Abziehen des Lösungsmittels im Vakuum erhält man 2-(2',2'-Dichlorvinyl)-3',3'-dimethyl-cyclopropyl)-oxazolin vom $Kp_{1mm}$ 86 bis 88°C.

## Beispiel 2

Herstellung von 2-(2,2-Dimethylvinyl)-3,3-dimethylcyclopropanthiocarbonsäure-N-t-butylamid

2.1 9,15 g (10 mmol) 3,3-Dimethyl-4-pentensäure-N-tert.-butylamid vom Fp. 73 bis 75°C (s. Beispiel 3a) werden mit 5 g Phosphorpentasulfid in 100 ml Pyridin 1 Stunde unter Rückfluß erhitzt. Die Lösung wird filtriert und Pyridin im Vakuum abdestilliert. Der Rückstand wird in Tetrachlorkohlenstoff aufgenommen.

2.2 Diese Lösung wird bei sukzessiver Zugabe von 0,5 g Dibenzoylperoxid 12 Stunden unter Rückfluß erhitzt. Tetrachlorkohlenstoff wird im Vakuum abgezogen und der Rückstand in 100 ml tert.-Butanol gelöst.

2.3 Unter Zusatz von 17 g (0,15 Mol) Kalium-t-butylat erhitzt man die Lösung 2 Stunden unter Rückfluß. Nach dem Erkalten verdünnt man das Reaktionsgemisch mit Methylenchlorid und schüttelt mit Wasser gut aus. Die organische Phase wird getrocknet und im Vakuum abgezogen. Zurück bleibt 2-(2,2-Dimethylvinyl)-3,3-dimethyl-cyclopropan-thiocarbonsäure-N-t-butylamid.

## Beispiel 3

Herstellung von 2-(2,2-Dichlorvinyl)-3,3-dimethylcyclopropancarbonsäure-amidoxim

3.1 4 g (0,12 Mol) Hydroxylamin in 100 ml n-Butanol werden mit 10,9 g (0,1 Mol) 4-Cyano-3,3-dimethyl-1-buten 48 Stunden bei 40°C gerührt. Dann wird n-Butanol im Vakuum abdestilliert und der Rückstand in Tetrachlorkohlenstoff gelöst.

3.2 Diese Lösung wird unter periodischem Zusatz von insgesamt 0,5 g Dibenzoylperoxid 12 Stunden unter Rückfluß erhitzt. Dann wird das Lösungsmittel abgezogen und der Rückstand in Ethanol aufgenommen.

3.3 Die ethanolische Lösung wird nach Zusatz von 0,3 Mol Natriumethylat 3 Stunden unter Rückfluß erhitzt. Dann wird die Lösung im Vakuum eingeengt, mit Ether verdünnt und mit Wasser gut

ausgeschüttelt. Nach dem Trocknen und Abdampfen des Ethers erhält man 2-(2,2-Dichlorvinyl)-3,3-dimethylcyclopropancarbonsäure-amidoxim.

Beispiel 4

Herstellung von 2-(2,2-Dichlorvinyl)-3,3-dimethyl-cyclopropancarbonhydroxamsäure

4.1 12,8 g (0,1 Mol) 3,3-Dimethyl-4-pentensäure (J.Org. Chem. *27*, 3602 (1962)) werden durch Kochen mit 50 ml Ethanol unter Zusatz einer Spur p-Toluolsulfonsäure in 8 Stunden azeotrop verestert. Dann wird eine Lösung von 3,3 g Hydroxylamin in 20 ml Ethanol sowie 0,01 Mol Natriumethylat zugegeben und 24 Stunden bei RT gerührt. Die Lösung wird mit verd. Salzsäure neutralisiert und im Vakuum eingeengt. Mit Tetrachlorkohlenstoff extrahiert man den Rückstand.

4.2 Diese Lösung wird bei sukzessiver Zugabe von 0,5 g Benzoylperoxid 15 Stunden am Rückfluß erhitzt. Dann wird das Lösungsmittel abgezogen und der Rückstand in Ethanol gelöst.

4.3 Man gibt 0,3 Mol Natriumethylat zu dieser Lösung und erhitzt 4 Stunden unter Rückfluß. Dann engt man im Vakuum ein, stellt mit verdünnter Salzsäure sauer und extrahiert mit Ether. Die Etherlösung wird nach Waschen mit Wasser getrocknet und einrotiert. Zurück bleibt 2-(2,2-Dichlorvinyl)-3,3-dimethylcyclopropanhydroxamsäure.

**Patentansprüche**

1. Derivate der 2-(2,2-Dihalogenvinyl)-3,3-dimethylcyclopropancarbonsäure der allgemeinen Formel I

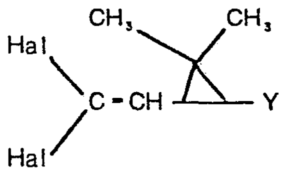

(I)

in welcher

Hal unabhängig voneinander für Halogen steht und

Y für ein C-Atom steht, das ausschließlich sowohl einfach als auch doppelt an Sauerstoff oder Schwefel und/oder Stickstoff gebunden ist, wobei mindestens eine Bindung durch Stickstoff besetzt ist und Y nicht für den Carbonamidrest steht.

2. Verfahren zur Herstellung der Derivate der 2-(2,2-Dihalogenvinyl)-3,3-dimethylcyclopropancarbonsäure der allgemeinen Formel I gemäß Anspruch 1, dadurch gekennzeichnet, daß man eine Verbindung der allgemeinen Formel II

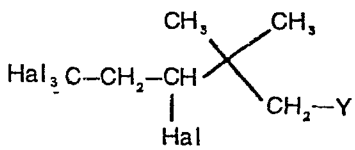

(II)

in welcher

Hal und Y die oben angegebene Bedeutung haben, in Gegenwart eines Verdünnungsmittels mit einer Base behandelt.

3. Verbindungen der allgemeinen Formel II gemäß Anspruch 2, wenn sie im Verfahren gemäß Anspruch 2 angewendet werden.

4. Verfahren zur Herstellung der Verbindungen II gemäß Anspruch 3, dadurch gekennzeichnet, daß man Verbindungen der Formel III

$$CH_2{=}CH{-}C(CH_3)_2{-}CH_2{-}Y$$

in welcher

Y die in Anspruch 1 angegebene Bedeutung hat, gegebenenfalls in Gegenwart eines Verdünnungsmittels mit Tetrahalogenmethan in Gegenwart eines Katalysators umsetzt.

5. Verbindungen der allgemeinen Formel III

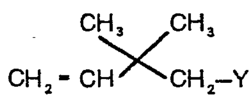

(III)

in welcher

Y für den Oxazolinrest der allgemeinen Formel IV steht

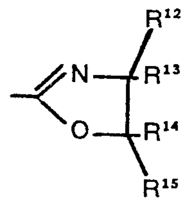

(IV)

in welcher

$R^{12}$—$R^{15}$ unabhängig voneinander für Wasserstoff, Alkyl, Aralkyl, Aryl stehen oder gemeinsam mit den angrenzenden C-Atomen einen carocyclishcen Ring bilden können, wenn sie im Verfahren gemäß Anspruch 4 zur Herstellung der Verbindungen gemäß Anspruch 3 angewendet werden.

6. Verfahren zur Herstellung der Verbindungen der allgemeinen Formel III gemäß Anspruch 5, dadurch gekennzeichnet, daß man 4-Cyano-3,3-dimethyl-1-buten der Formel V

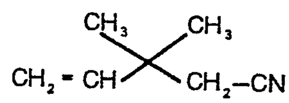

(V)

mit 2-Amino-alkanolen der allgemeinen Formel VI

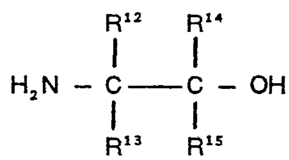

(VI)

in welcher

$R^{12}$—$R^{15}$ die oben angegebene Bedeutung haben, bei Temperaturen von 150 bis 200°C gegebenenfalls in Gegenwart eines Katalysators sowie eines Verdünnungsmittels umsetzt.

**Revendications**

1. Dérivés de l'acide 2-(2,2-dihalogénovinyl)-3,3-diméthylcyclopropanecarboxylique de formule générale I

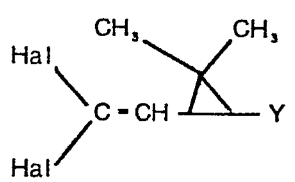

(I)

dans laquelle

Hal représente chacun indépendamment un halogène, et

Y représente un atome de carbone qui est exclusivement relié par une liaison simple ou également une liaison double à l'oxygène ou au soufre et/ou à l'azote, au moins une liaison étant occupée par l'azote, et Y n'est pas le reste carboxamide.

2. Procédé pour la préparation des dérivés de l'acide 2-(2,2-dihalogénovinyl)-3,3-diméthylcyclopropanecarboxylique de formule générale I selon la revendication 1, caractérisé en ce que l'on traite un composé de formule générale

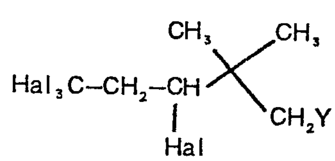

(II)

dans laquelle

Hal et Y sont tels que définis ci-dessus par une base en présence d'un agent diluant.

3. Composés de formule générale II selon la revendication 2.

4. Procédé pour la préparation des composés II selon la revendication 3, caractérisé en ce que l'on fait réagir des composés de formule III

$$CH_2{=}CH{-}C(CH_3)_2{-}CH_2{-}Y$$

dans laquelle Y est tel que défini ci-dessus avec un tétrahalogénométhane, éventuellement en présence d'un agent diluant, en présence d'un catalyseur.

5. Composés de formule générale III

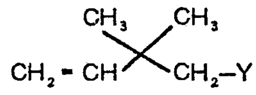

(III)

dans laquelle Y représente un reste oxazoline de formule générale (IV)

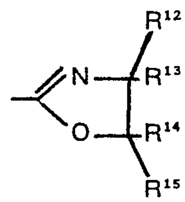

(IV)

dans laquelle

$R^{12}$ à $R^{15}$ représentent indépendamment l'un de l'autre l'hydrogène ou un groupe alkyle, arylalkyle, aryle ou peuvent former ensemble avec les atomes de carbone qui les limitent un noyau carboxylique, si on les utilise dans le procédé selon la revendication 4, pour la préparation des composés selon la revendication 3.

6. Procédé pour la préparation des composés de formule générale III selon la revendication 5, caractérisé en ce que l'on fait réagir le 4-cyano-3,3-diméthyl-1-butène de formule (V)

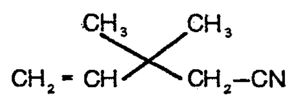

(V)

avec des 2-aminoalcanols de formule générale (VI)

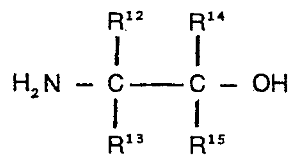

(VI)

dans laquelle

$R^{12}$—$R^{15}$ sont tels que définis ci-dessus, à des températures de 150 à 200°C, éventuellement en présence d'un catalyseur ainsi que d'un agent diluant.

**Claims**

1. Derivatives of 2-(2,2-dihalogenovinyl)-3,3-dimethylcyclopropanecarboxylic acid of the general formula I

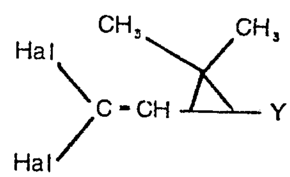

(I)

in which

the Hal's independently of one another represent halogen and

Y represents a C-atom which is exclusively linked both by a single bond and by a double bond to oxygen or sulphur and/or nitrogen, at least one bond being taken up by nitrogen and Y does not represent the carboxylic amide radical.

2. A process for the preparation of the derivatives of 2-(2,2-dihalogenovinyl)-3,3-dimethylcyclopropanecarboxylic acid of the general formula I according to claim 1, characterised in that a compound of the general formula II

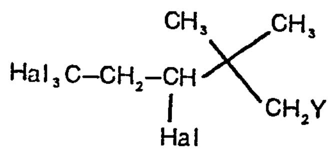

(II)

in which

Hal and Y have the meaning indicated above, is treated with a base in the presence of a diluent.

3. Compounds of the general formula II according to claim 2 when they are used in the process according to claim 2.

4. A process for the preparation of the compounds II according to claim 3, characterised in that compounds of the formula III

$$CH_2{=}CH{-}C(CH_3)_2{-}CH_2{-}Y$$

in which

Y has the meaning indicated in claim 1, are reacted with tetrahalogenomethane optionally in the presence of a diluent and in the presence of a catalyst.

5. Compounds of the general formula III

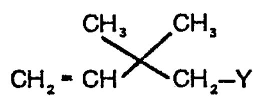

(III)

in which

Y represents the oxazoline radical of the general formula IV

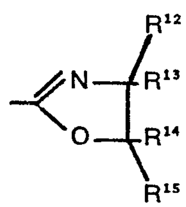

(IV)

in which

$R^{12}$—$R^{15}$, independently of one another represent hydrogen, alkyl, aralkyl, aryl or together with the adjacent C-atoms can form a carbocyclic ring, when they are used in the process according to claim 4 for the preparation of the compounds according to claim 3.

6. A process for the preparation of the compounds of the general formula III according to claim 5, characterised in that 4-cyano-3,3-dimethyl-1-butene of the formula V

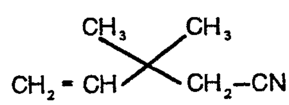

(V)

is reacted with 2-amino-alkanoles of the general formula VI

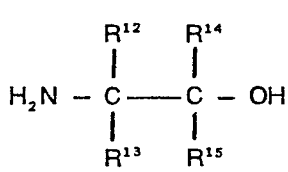

(VI)

in which

$R^{12}$—$R^{15}$ have the meaning indicated above, at temperatures of 150 to 200°C optionally in the presence of a catalyst and a diluent.